# EUROPEAN PATENT SPECIFICATION

(11) **EP 1 328 303 B1**
(45) Date of publication and mention of the grant of the patent: **24.11.2004**
(21) Application number: 01981333.6
(22) Date of filing: 27.09.2001
(51) Int. Cl.: A61L 27/34, A61L 31/10, A61L 29/08

(54) **PREVENTION OF BACTERIAL ATTACHMENT TO BIOMATERIALS BY CATIONIC POLYSACCHARIDES**
VERWENDUNG VON KATIONISCHEN POLYSACCHARIDEN ZUR VERHINDERUNG VON BAKTERIENANSÄTZEN
PREVENTION DE LA FIXATION BACTERIENNE SUR DES BIOMATERIAUX AU MOYEN DE POLYSACCHARIDES CATIONIQUES

(30) Priority: 24.10.2000 US 695529
(43) Date of publication of application: 23.07.2003
(73) Proprietor: BAUSCH & LOMB INCORPORATED, Rochester, New York 14604-2701 (US)
(72) Inventor: BORAZJANI, Roya, Rochester, NY 14618 (US); SALAMONE, Joseph, C, Boca Raton, FL 33486 (US); AMMON, JR, Daniel, M, Rochester, NY 14609 (US); KUNZLER, Jay, F, Canandaigua, NY 14424 (US); HU, Zhenze, Pittsford, NY 14534 (US)
(74) Representative: Bowman, Paul Alan
(86) International application number: PCT/US2001/030373
(87) International publication number: WO 2002/034308

(56) References cited:
- WO-A-00/37048
- WO-A-95/00618
- US-A- 5 328 954

## Description

### FIELD OF THE INVENTION

The present invention is directed to the surface treatment of medical devices including ophthalmic lenses, stents, implants and catheters. In particular, the present invention is directed to a simple, low cost method of modifying the surface of a medical device to decrease its affinity for bacterial adhesion.

### BACKGROUND

Medical devices such as ophthalmic lenses have been investigated for a number of years. Such materials can generally be subdivided into two major classes, namely hydrogels and non-hydrogels. Non-hydrogels do not absorb appreciable amounts of water, whereas hydrogels can absorb and retain water in an equilibrium state.

Those skilled in the art have long recognized that surface characteristics play a major role in biocompatibility. It is known that increasing the hydrophilicity of the contact lens surface improves the wettability of the contact lenses. This in turn is associated with improved wear comfort of contact lenses. Additionally, the surface of the lens can affect the lens's susceptibility to deposition, particularly the deposition of proteins and lipids from the tear fluid during lens wear. Accumulated deposition can cause eye discomfort or even inflammation. In the case of extended wear lenses (i.e. lenses used without daily removal of the lens before sleep), the surface is especially important, since extended wear lenses must be designed for high standards of comfort and biocompatibility over an extended period of time.

Extended-wear lenses also present two added challenges. First, the lenses are typically in continuous contact with the epithelium for between 7 and 30 days. This stands in marked contrast to conventional contact lenses, which are removed from the eye before sleep. Second, because the extended-wear lenses are worn continuously, they are generally not removed for disinfection until the conclusion of the recommended extended-wear period. Thus an improved method for inhibiting bacterial attachment would be a major advance for both conventional and extended-wear contact lenses.

In the area of contact lens wetting/conditioning solutions, it has been found that polyelectrolytes can bind to a lens surface of opposite charge and form polyelectrolyte complexes. Such polyelectrolyte complexes have commercially been demonstrated to give more comfortable lens materials because of the greater adsorption of surface bound water. Examples of materials useful for forming such polyelectrolyte complexes are taught in U.S. Patents 4,321,261 to Ellis et al.; 4,436,730 to Ellis et al.; 5,401,327 to Ellis et al.; 5,405,878 to Ellis et al.; 5,500,144 to Potini et al.; 5,604,189 to Zhang et al.; 5,711,823 to Ellis et al.; 5,773,396 to Zhang et aL; and 5,872,086 to Ellis et al.

Bacterial attachment to biomaterial surfaces is believed to be a contributing factor in device-related infection. But the extent to which a given microorganism will attach itself to a given biomaterial has proven difficult to predict Examples of methods for inhibiting such attachment are taught in U.S. Patents 5,945,153 to Dearnaley; 5,961,958 to Homola et al.; 5,980,868 to Homola et aL; 5,984,905 to Dearnaley; 6,001,823 to Hultgren et al.; 6,013,106 to Tweden et al.; and 6,054,054 to Robertson et al.

For contact lens materials, bacterial attachment to a lens surface can lead to bacterial keratitis, or other potential contact lens related complications such as sterile infiltrates and CLARE (Contact Lens Induced Acute Red Eye). Thus it would be desirable to provide a method for inhibiting attachment of microorganisms to contact lenses.

WO00/37048 discloses a method for treating a silicone-hydrogel contact lens while worn in the eye which employs an ophthalmic solution containing a cationic cellulose polymer that binds to the lens and prevents the accumulation of lipids, proteins and other products, particularly during extended use of the lens.

The present invention uses a cationic polysaccharide to inhibit adhesion of bacteria to the surface of a biomedical device by binding the cationic polysaccharide to the surface of the biomedical device.

The invention also relates to the use of a cationic polysaccharide for the manufacture of a composition for inhibiting adhesion of bacteria to the surface of a biomedical device.

The surface of the biomaterial, is preferably at least slightly anionic prior to the application of the cationic polysaccharide. The mechanism for binding the cationic polysaccharide to the surface of the biomedical device is not critical, provided that the binding strength is sufficient to maintain the surface for the intended use of the biomaterial. As used herein, the terms "bond" and "bind" refer to forming a relatively stable complex or other relatively stable attraction between the surface of a biomedical device and a polysaccharide with or without the addition of a linking agent, and is not limited to a particular mechanism. Thus "binding" may involve covalent bonds, hydrogen bonds, hydrophobic interactions or other molecular interactions that enable the cationic polysaccharide of the invention to form a relatively tenacious surface coating on a biomedical device.

The cationic charge on the cationic polysaccharide may be derived from ammonium groups, quaternary ammonium groups, guanidium groups, sulfonium groups, phosphonium groups, bound transition metals, and other positively charged functional groups.

Examples of methods for providing an anionic surface charge on the biomedical device include: (a) bulk distribution of anionic sites in the biomaterial for example, by polymerization; (b) oxidative surface treatment such as plasma discharge or corona discharge; (c) application of an anionic linking agent; (d) complexation; or (e) a combination of one or more of (a) - (d).

Incorporating monomers containing groups such as carboxylate groups, sulfate groups, sulfonate groups, sulfite groups, phosphate groups, phosphonate groups, and phosphonic groups can provide anionic sites distributed through the bulk of the polymeric substrate material. Methacrylic acid and 2-acrylamido-2-methylpropane sulfonic acid are examples of monomers that are useful for incorporating negatively charged sites into the bulk of the substrate biomaterial.

If the surface of the biomaterial carries a net neutral charge or a net cationic charge, the biomaterial may be treated with an oxidative surface treatment or other surface treatment to present a net anionic charge prior to the treatment with the cationic polysaccharide. Examples of suitable oxidative surface treatments include plasma discharge or corona discharge as taught in U.S. Patents 4,217,038 to Letter; 4,096,315 to Kubacki; 4,312,575 to Peyman; 4,631,435 to Yanighara; and 5,153,072; 5,091,204 and 4,565,083, all to Ratner. Additional examples of plasma surface treatments include subjecting contact lens surfaces to a plasma comprising an inert gas or oxygen (see, for example, U.S. Patent Nos. 4,055,378; 4,122,942; and 4,214,014); various hydrocarbon monomers (see, for example, U.S. Patent No. 4,143,949); and combinations of oxidizing agents and hydrocarbons such as water and ethanol (see, for example, WO 95/04609 and U.S. Patent No 4,632,844).

The cationic polysaccharide may attach to the surface of the biomaterial through interactions between hydrophobic sites on the biomaterial surface interacting with hydrophobic groups on the cationic polysaccharide. Covalent linkages may also exist between the surface of the biomaterial and the water-soluble cationic polysaccharide such that the cationic polysaccharide is bound to the biomaterial surface.

The cationic polysaccharide may also bind to the surface of the biomedical device through hydrogen-bonding interactions. These hydrogen-bonding interactions may occur between hydrogen-bond accepting surfaces and hydrogen-bond donating solutions, or between hydrogen-bond donating surfaces and hydrogen-bond accepting solutions. Examples of hydrogen-bond accepting groups include pyrrolidone groups, acrylamide groups, polyether groups and fluorocarbon groups. Examples of suitable polyether groups include poly(ethylene glycol) or poly(ethylene oxide). Examples of suitable hydrogen-donating groups include carboxylic acids, sulfuric acids, sulfonic acids, sulfinic acids, phosphoric acids, phosphonic acids, phosphinic acids, phenolic groups, hydroxy groups, amino groups and imino groups.

Examples of linkages include those provided by coupling agents such as ester linkages and amide linkages. Surface linkages may also include surface complexations. Examples of such surface complexations include the reaction products formed by treating a biomaterial comprising a hydrophilic monomer and a silicone-containing monomer with a proton-donating wetting agent, where the wetting agent forms a complex with hydrophilic monomer on the surface of the biomaterial in the absence of a surface oxidation treatment step.

The biomedical device may be an ophthalmic lens, for example an intraocular lens, a contact lens or a corneal inlay. The biomedical device may also be a contact lens case, more particularly the interior portion of a contact lens case. The method of the invention is useful with soft lens materials such as hydrogels as well as with rigid contact lens materials. The method of the invention is especially useful with extended-wear contact lenses that are suitable for periods of continuous wear for about 7 to about 30 days.

The cationic cellulosic polymers of the invention have been found to exhibit strong anti-attachment properties (activity) for the bacterium, *Pseudomonus aeruginosa*, as shown in studies of attachment to contact lens surfaces. Examples of useful cationic polysaccharides are derived from the families based on cellulosics, guar gum, starch, dextran, chitosan, locust bean gum, gum tragacanth, curdlan, pullulan and seleroglucan. Of particular interest are the cationic polymers derived from cellulosic materials. It is believed that the degree of inhibition activity is related to the strength of the ionic bonding between the polymeric surface coating and the lens surface. Thus, independent of the mechanism, stronger bonds are believed to be associated with a greater degree of resistance to bacterial adhesion.

### BRIEF DESCRIPTION OF DRAWINGS

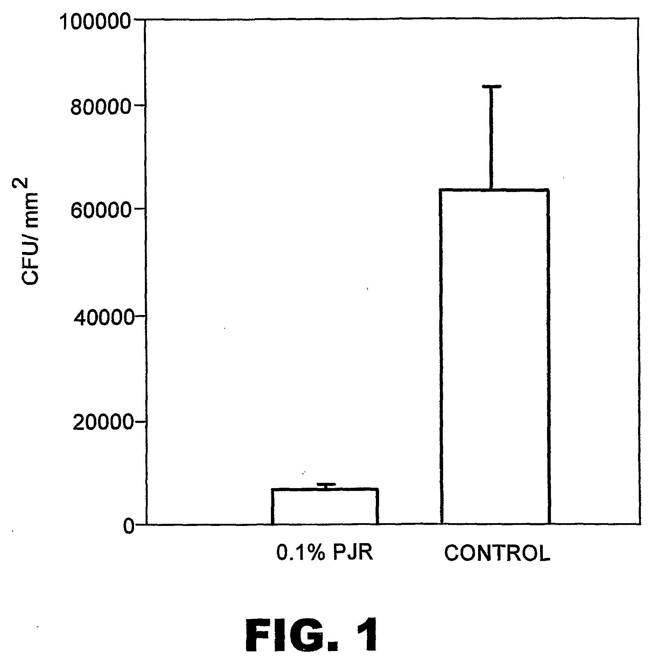

FIG. 1 shows the results of Example 3, comparing the concentration of bacterium *Pseudomonas aeruginosa* on the surface of an extended-wear hydrogel contact lens with and without a surface coating of a cationic cellulosic polymer applied in a two-minute soaking step.

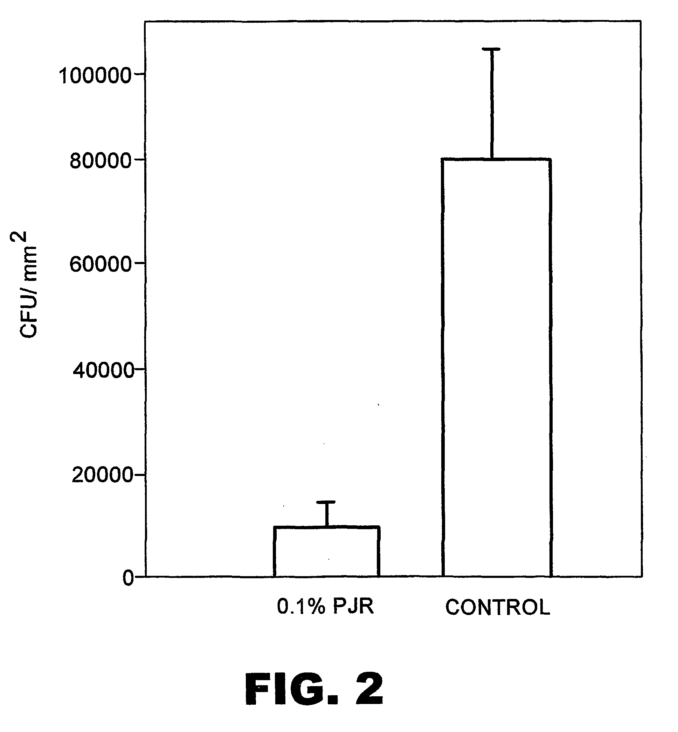

FIG. 2 shows the results of Example 4, comparing the concentration of bacterium *Pseudomonas aeruginosa* on the surface of an extended-wear hydrogel contact lens with and without a surface coating of a cationic cellulosic polymer applied in a four-hour soaking step.

### DETAILED DESCRIPTION OF THE INVENTION

The invention is applicable to a wide variety of biomaterials, including ophthalmic lens materials as mentioned above. Examples of ophthalmic lenses include contact lenses, anterior and posterior chamber lenses, intraocular lenses and corneal inlays. Ophthalmic lenses may be fabricated from flexible or rigid materials, depending upon the characteristics needed for a particular application.

### Substrate Materials

Hydrogels comprise hydrated, crosslinked polymeric systems containing water in an equilibrium state. Conventional hydrogel lens materials include polymers containing monomers such as 2-hydroxyethyl methacrylate (HEMA), glyceryl methacrylate, N-vinylpyrrolidone (NVP) and dimethacrylamide.

Flexible ophthalmic lens materials useful in the present invention include silicone hydrogels as well as conventional hydrogels and low-water elastomeric materials. Examples of flexible ophthalmic lens materials useful in the present invention are taught in U.S. Patents 5,908,906 to Künzler et al.; 5,714,557 to Künzler et al.; 5,710,302 to Künzler et al.; 5,708,094 to Lai et al.; 5,616,757 to Bambury et al.; 5,610,252 to Bambury et al.; 5,512,205 to Lai; 5,449,729 to Lai; 5,387,662 to Künzler et al. and 5,310,779 to Lai.

U.S. Patents 6,037,328, 6,008,317, 5,981,675, 5,981,669, 5,969,076, 5,945,465, 5,914,355, 5,858,937, 5,824,719 and 5,726,733 teach ophthalmic lens materials containing HEMA monomers.

U.S. Patents 6,071,439, 5,824,719, 5,726,733, 5,708,094, 5,610,204, 5,298,533, 5,270,418, 5,236,969 and 5,006,622 teach ophthalmic lens materials containing glyceryl methacrylate monomers.

U.S. Patents 6,008,317, 5,969,076, 5,908,906, 5,824,719, 5,726,733, 5,714,557, 5,710,302, 5,708,094, 5,648,515 and 5,639,908 teach ophthalmic lens materials containing NVP monomers.

U.S. Patents 5,539,016, 5,512,205, 5,449,729, 5,387,662, 5,321,108 and 5,310,779, teach ophthalmic lens materials containing dimethacrylamide monomers.

The preferred conventional hydrogel materials typically contain HEMA, NVP and TBE (4-t-butyl-2-hydroxycyclohexyl methacrylate). Polymacon™ materials, for example the Softens 66™ brand contact lenses (commercially available from Bausch & Lomb Incorporated of Rochester, New York), are examples of particularly preferred conventional hydrogel materials.

Silicone hydrogels generally have a water content greater than about five weight percent and more commonly between about ten to about eighty weight percent. Materials are usually prepared by polymerizing a mixture containing at least one silicone-containing monomer and at least one hydrophilic monomer. Either the silicone-containing monomer or the hydrophilic monomer may function as a crosslinking agent (a crosslinker being defined as a monomer having multiple polymerizable functionalities) or a separate crosslinker may be employed. Applicable silicone-containing monomeric units for use in the formation of silicone hydrogels are well known in the art and numerous examples are provided in U.S. Patent Nos. 4,136,250; 4,153,641; 4,740,533; 5,034,461; 5,070,215; 5,260,000; 5,310,779; and 5,358,995.

A preferred silicone hydrogel material comprises (in the bulk monomer mixture that is copolymerized) 5 to 50 percent, preferably 10 to 25, by weight of one or more silicone macromonomers, 5 to 75 percent, preferably 30 to 60 percent, by weight of one or more polysiloxanylalkyl (meth)acrylic monomers, and 10 to 50 percent, preferably 20 to 40 percent, by weight of a hydrophilic monomer. In general, the silicone macromonomer is a poly(organosiloxane) capped with an unsaturated group at two or more ends of the molecule. In addition to the end groups in the above structural formulas, U.S. Patent No. 4,153,641 to Deichert et al. discloses additional unsaturated groups, including acryloxy or methacryloxy. Fumarate-containing materials such as those taught in U.S. Patents 5,512,205; 5,449,729; and 5,310,779 to Lai are also useful substrates in accordance with the invention. Preferably, the silane macromonomer is a silicon-containing vinyl carbonate or vinyl carbamate or a polyurethane-polysiloxane having one or more hard-soft-hard blocks and end-capped with a hydrophilic monomer.

Suitable hydrophilic monomers include those monomers that, once polymerized, can form a complex with poly(acrylic acid). The suitable monomers form hydrogels useful in the present invention and include, for example, monomers that form complexes with poly(acrylic acid) and its derivatives. Examples of useful monomers include amides such as N,N-dimethyl acrylamide, N,N-dimethyl methacrylamide, cyclic lactams such as N-vinyl-2-pyrrolidone and poly(alkene glycol)s functionalized with polymerizable groups. Examples of useful functionalized poly(alkene glycol)s include poly(diethylene glycol)s of varying chain length containing monomethacrylate or dimethacrylate end caps. In a preferred embodiment, the poly(alkene glycol) polymer contains at least two alkene glycol monomeric units. Still further examples are the hydrophilic vinyl carbonate or vinyl carbamate monomers disclosed in U.S. Patent Nos. 5,070,215, and the hydrophilic oxazolone monomers disclosed in U.S. Patent No. 4,910,277. Other suitable hydrophilic monomers will be apparent to one skilled in the art. In a particularly preferred embodiment, the hydrophilic monomers used in the contact lens material are capable of forming a stable complex with a cationic polysaccharide.

Rigid ophthalmic lens materials include rigid-gas-permeable ("RGP") materials. RGP materials typically comprise a hydrophobic crosslinked polymer system containing less than 5 wt. % water. RGP materials useful in accordance with the present invention include those materials taught in US Patent No. 4,826,936 to Ellis; 4,463,149 to Ellis; 4,604,479 to Ellis; 4,686,267 to Ellis et al.; 4,826,936 to Ellis; 4,996,275 to Ellis et al.; 5,032,658 to Baron et al.; 5,070,215 to Bambury et al.; 5,177,165 to Valint et al.; 5,177,168 to Baron et al.; 5,219,965 to Valint et al.; 5,336,797 to McGee and Valint; 5,358,995 to Lai et al.; 5,364,918 to Valint et al.; 5,610,252 to Bambury et al.; 5,708,094 to Lai et al; and 5,981,669 to Valint et al. US Patent 5,346,976 to Ellis et al. teaches a preferred method of making an RGP material.

Other non-silicone hydrogels used for extended wear applications are also applicable, provided that surface attachment of the cationic polysaccharide can be achieved. The method of the invention is also useful for treating biomaterials before or after fabrication as a broad range of medical devices including intraocular lenses, artificial corneas, stents and catheters, merely to name a few examples.

### Surface Coating Materials

Surface coating materials useful in the present invention include cationic polysaccharides, for example cationic cellulosic polymers. Specific examples include cellulosic polymers containing N,N-dimethylaminoethyl groups (either protonated or quaternized) and cellulosic polymers containing N,N-dimethylamino-2-hydroxylpropyl groups (either protonated or quaternized). Cationic cellulosic polymers are commercially available or can be prepared by methods known in the art. As an example, quaternary nitrogen-containing ethoxylated glucosides can be prepared by reacting hydroxyethyl cellulose with a trimethylammonium-substituted epoxide. Various preferred cationic cellulosic polymers are commercially available, for example water-soluble polymers available under the CTFA (Cosmetic, Toiletry, and Fragrance Association) designation Polyquaternium-10. Such polymers are commercially available under the tradename UCARE® Polymer from Amerchol Corp., Edison, NJ, USA. These polymers contain quaternized N,N-dimethylamino groups along the cellulosic polymer chain. The cationic cellulosic component may be employed in the compositions at about 0.01 to about ten (10) weight percent of the composition, preferably at about 0.05 to about five (5) weight percent, with about 0.1 to about one (1) weight percent being especially preferred. Suitable cationic cellulosic materials have the following formula: 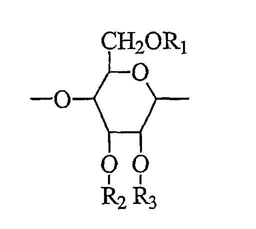 Wherein R₁ R₂ and R₃ are selected from H, derivatives of C₁-C₂₀ carboxylic acid, C₁-C₂₀ alkyl groups, C₁ to C₃ monohydric and dihydric alkanols, hydroxyethyl groups, hydroxypropyl groups, ethylene oxide groups, propylene oxide groups, phenyl groups, "Z" groups and combinations thereof. At least one of R₁, R₂,and R₃ is a Z group.

The nature of the "Z" groups is: 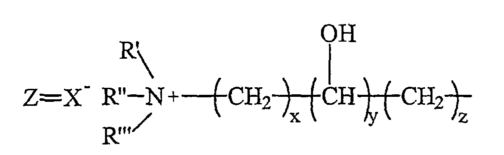 where: 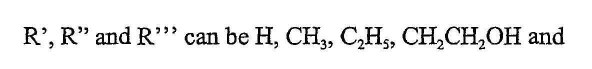 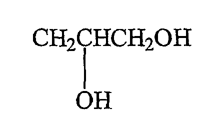 x=0-5, y=0-4, and z=0-5
X⁻ = Cl⁻, Br⁻, I⁻, HSO₄⁻, CH₃SO₄⁻, H₂PO₄⁻, NO₃⁻

U.S. Patent No. 5,645,827 to Marlin, et al. discloses the use of compositions comprising a cationic polysaccharide in combination with an anionic therapeutic agent, for example, hyaluronic acid or its salt, which is a known demulcent for the treatment of dry eye. European Application 088770 A1 to Marlin et al. discloses cationic cellulose polymers to deliver cationic therapeutic agents, especially for the treatment of glaucoma.

U.S. Patent Nos. 4,436,730 and 5,401,327 to Ellis, et al. disclose the use of cationic cellulosic derivatives in contact-lens treating solutions, including the combination of a cationic cellulose polymer and an ethoxylated glucose such as glucam.

Optionally, one or more additional polymeric or non-polymeric demulcents may be combined with the above-named ingredients. Demulcents are known to provide wetting, moisturizing and/or lubricating effects, resulting in increased comfort. Polymeric demulcents can also act as a water-soluble viscosity builder. Included among the water-soluble viscosity builders are the non-ionic cellulosic polymers like methyl cellulose, hydroxyethyl cellulose, hydroxypropyl cellulose, hydroxypropyl methylcellulose and carboxymethyl cellulose, poly(N-vinylpyrrolidone), poly(vinyl alcohol) and the like. Such viscosity builders or demulcents may be employed in a total amount ranging from about 0.01 to about 5.0 weight percent or less. The viscosity of the final formulation ranges from 2 centipoise (cps) to several million cps depending upon whether the formulation is intended for contact lenses, intraocular lenses or corneal inlays. Comfort agents such as glycerin or propylene glycol can also be added.

The present composition may also contain a disinfecting amount of a preservative or an antimicrobial agent. The presence of an antimicrobial agent is not required; however, for the invention to reduce effectively the concentration of bacteria on the surface of a biomaterial, a particularly preferred preservative is sorbic acid (0.15%). Antimicrobial agents are defined as organic chemicals that derive their antimicrobial activity through a chemical or physiochemical interaction with the microbial organisms. For example, biguanides include the free bases or salts of alexidine, chlorhexidine, hexamethylene biguanides and their polymers, and combinations of the foregoing. The salts of alexidine and chlorhexidine can be either organic or inorganic and are typically gluconates, nitrates, acetates, phosphates, sulfates, halides and the like. The preferred biguanide is the hexamethylene biguanide commercially available from Zeneca, Wilmington, DE under the trademark Cosmocil™ CQ. Generally, the hexamethylene biguanide polymers, also referred to as polyaminopropyl biguanide (PAPB), have molecular weights of up to about 100,000.

If used in the subject solution, the antimicrobial agent should be used in an amount that will at least partially reduce the microorganism population in the formulations employed. Preferably, a disinfecting amount is that which will reduce the microbial bioburden by two log orders in four hours and more preferably by one log order in one hour. Most preferably, a disinfecting amount is an amount which will eliminate the microbial burden on a contact lens when used in regimen for the recommended soaking time (FDA Chemical Disinfection Efficacy Test-July, 1985 Contact Lens Solution Draft Guidelines). Typically, such agents are present in concentrations ranging from about 0.00001 to about 0.5% (w/v), and more preferably, from about 0.00003 to about 0.05% (w/v).

The aqueous solutions employed in this invention may contain, in addition to the active ingredients described above, one or more other components that are commonly present in ophthalmic solutions, for example, buffers, stabilizers, tonicity agents and the like, which aid in making ophthalmic compositions more comfortable to the user. The aqueous solutions of the present invention are typically adjusted with tonicity agents to approximate the tonicity of normal lacrimal fluids which is equivalent to a 0.9% solution of sodium chloride or 2.8% of glycerol solution. The solutions are made substantially isotonic with physiological saline used alone or in combination; otherwise, if simply blended with sterile water and made hypotonic or made hypertonic, the lenses will lose their desirable optical parameters. Correspondingly, excess salt or other tonicity agents may result in the formation of a hypertonic solution that will cause stinging and eye irritation. An osmolality of about 225 to 400 mOsm/kg is preferred, more preferably 280 to 320 mOsm/kg.

The pH of the present solutions should be maintained within the range of 5.0 to 8.0, more preferably about 6.0 to 8.0, most preferably about 6.5 to 7.8; suitable buffers may be added, such as borate, citrate, bicarbonate, TRIS and various mixed phosphate buffers (including combinations of Na₂HPO₄, NaH₂PO₄ and KH₂PO₄) and mixtures thereof. Borate buffers are preferred, particularly for enhancing the efficacy of PAPB. Generally, buffers will be used in amounts ranging from about 0.05 to 2.5 percent by weight, and preferably, from 0.1 to 1.5 percent.

In addition to buffering agents, in some instances it may be desirable to include sequestering agents in the present solutions in order to bind metal ions, which might otherwise react with the lens and/or protein deposits and collect on the lens. Ethylenediaminetetraacetic acid (EDTA) and its salts (disodium) are preferred examples. They are usually added in amounts ranging from about 0.01 to about 0.2 weight percent.

The solutions employed in the present invention can be prepared by a variety of techniques. One method employs two-phase compounding procedures. In the first phase, about 30 percent of the distilled water is used to dissolve the cationic cellulosic polymer by mixing for about 30 minutes at around 50° C. The first-phase solution is then autoclaved at about 120° C for 30 minutes. In a second phase, alkali metal chlorides, sequestering agents, preservatives and buffering agents are then dissolved in about 60 percent of the distilled water under agitation, followed by the balance of distilled water. The second-phase solution can then be sterilely added into the first-phase solution by forcing it through an 0.22 micron filter by means of pressure, followed by packaging in sterilized plastic containers.

As indicated above, the present invention is useful for improving comfort and wearability for extended-wear contact lenses. For that purpose, compositions for use in the present invention may be formulated as eye-drops and sold in a wide range of small-volume containers from 1 to 30 ml in size. Such containers can be made from HDPE (high density polyethylene), LDPE (low density polyethylene), polypropylene, poly(ethylene terepthalate) and the like. Flexible bottles having conventional eye-drop dispensing tops are especially suitable for use with the present invention. The eye-drop formulation of the invention used by instilling, for example, about one (1) or three (3) drops in the eye(s) as needed.

The present invention is also useful as a component of a cleaning, disinfecting or conditioning solution. The invention may also include antimicrobial agents, surfactants, toxicity adjusting agents, buffers and the like that are known to be useful components of conditioning and/or cleaning solutions for contact lenses. Examples of suitable formulations for cleaning and/or disinfecting solutions are taught in U.S. Patent 5,858,937 to Richard and Heiler.

### EXAMPLES

### Example 1

### Surface Conditioning of Surevue Lenses with Polymer JR

This example illustrates the binding effect of the cationic cellulosic polymer onto hydrophilic contact lenses, where it is believed to reduce the attachment of bacteria to the material surface. Three Surevue lenses (manufactured by Johnson & Johnson, New Brunswick, NJ) in three different solutions were submitted for comparison by Atomic Force Microscopy (AFM) analysis. Solution 1, for comparison, was a Blank Borate-Buffered Saline. Solution 2 was Solution 1 with 0.1% Polymer JR. Solution 3, for further comparison, was ReNu® MPS (manufactured by Bausch & Lomb, Rochester, NY). The lenses were treated overnight, and then removed from the vials and desalinated in HPLC grade water in a static fashion for a minimum of 15 minutes. All lenses were cut with a clean scalpel on a clean glass substrate. The samples were dried, sectioned and placed on a clean substrate. Three 50 x 50 µm topographical images were acquired for each side (anterior and posterior) of the lenses using AFM. The AFM used in this study was the Dimension 3000 and was operated in ContactMode. The AFM works by measuring nano-scale forces (10⁻⁹N) between a sharp probe and atoms on the lens surface. The resulting AFM images showed that the anterior and posterior surfaces of the lenses stored in Blank Borate-Buffered Saline (Solution 1) as well as ReNu@ MPS (Solution 3) showed no significant topographical change. The anterior and posterior surfaces of the lenses stored in Polymer JR solution (Solution 2) showed a significantly different topography. The surface is covered with a thin film, with multi-sized and shaped voids covering both anterior and posterior surfaces. These voids had an average depth of 40 ± 10 nm. These void-like anomalies were not present in the lenses stored in Solution 2 or Solution 3. The voids had an effect on the Root Mean Square (RMS) roughness for the lenses stored in the Polymer JR solution.

The RMS surface roughness was calculated using the Nanoscope software (shown in Table below). The lenses stored in Solution 1 or Solution 3 had a smoother anterior and posterior surface compared to the anterior and posterior of lenses stored in the Polymer JR solution.

**Table 1**

| RMS Roughness for Each Set of AFM Images | | | |
|---|---|---|---|
| Solution | Anterior | Posterior | Mean |
| Solution 1 | 3.93 nm | 3.03 nm | 3.48 nm |
| Solution 2 | 8.85 nm | 6.21 nm | 7.53 nm |
| Solution 3 | 5.82 nm | 3.09 nm | 4.46 nm |

The AFM results indicate that Polymer JR has an effect on the morphology of the lens surface, indicating a thin film covering with large multi-shaped and sized voids on the anterior and posterior side of the lens.

### Example 2

Aliquots of 20 ml of 0.1 % cationic Polymer JR solution were poured into sterile polystyrene disposable petri dishes. Negatively charged continuous wear lenses were removed from the packages with a sterile forceps and immersed five times in 180 ml of initially sterile 0.9% saline. These lenses were then placed into petri dishes containing 0.1% Polymer JR solutions and soaked for 4 h at room temperature. After 4 h incubation time, the ionically coated lenses were removed from the 0.1% Polymer JR solution with a sterile forceps and immersed 5 times in each of three successive changes (180ml) of initially sterile 0.9% saline. The lenses were then transferred to 20-ml glass scintillation vials containing 3 ml of ∼ 10⁸ cells/ml inoculum of radiolabeled cells and were incubated at 37°C for 2 h.

### Examples 3 and 4

Examples 3 and 4 evaluate bacterial adherence to biomaterials using a radiolabel method.

Adherence studies were conducted with a modification of the procedures of Sawant et al. (1) and Gabriel et al. (2). Bacterial cells were grown in Triptic Soy Broth (TSB) at 37°C on a rotary shaker for 12 to 18 h. Cells were harvested by centrifugation at 3000 x g for 10 min, washed two times in 0.9% saline and suspended in minimal medium(1.0g D-glucose, 7.0g K₂HPO₄, 2.0g KH₂PO₄, 0.5g sodium citrate, 1.0g (NH₄)₂SO₄, and 0.1g MgSO₄ in 1 liter distilled H₂O, pH 7.2) to a concentration of about ∼2 x10⁸ cells per ml (Optical density 0.10 at 600nm). The minimal broth cultures were incubated for 1 h at 37°C with shaking. One to 3 µCi/ml of L-[3,4,5-³H] leucine (NEN Research Products, Du Pont Company, Wilmington, DE) were added to the cells and the cell suspensions were incubated for another 20 min. These cells were washed 4 times in 0.9% saline and suspended in phosphate buffered saline (PBS) to a concentration of about ∼10⁸ cells per ml (Optical density 0.10 at 600nm).

Extended-wear contact lenses having a normally anionic surface charge were incubated with 3 ml of the radiolabeled cell suspension at 37°C for 2h. These lenses were removed from the cell suspension with a sterile forceps and immersed 5 times in each of three successive changes (180 ml) of initially sterile 0.9% saline. The lenses were shaken free from saline and transferred to 20-ml glass scintillation vials. Ten ml Opti-Fluor scintillation cocktail (Packard Instrument Co., Downers Grove, IL) were added to each vial. The vials were vortexed and then placed in a liquid scintillation counter (LS-7500, Beckman Instruments, Inc., Fullerton, CA). Data for two experiments were converted from disintegrations per min (dpm) to colony-forming units (cfu) based on a standard calibration curve and expressed as cfu/mm². Calibration curves were constructed from numbers of colonies recovered in pour plates of serial dilutions of inocula and from optical densities (O.D.s) of serial dilutions of cell suspensions of known densities. Uninoculated extended-wear contact lenses having normally anionic surface charge, which served as controls for the nonspecific uptake of leucine, were treated in the same manner as the inoculated sections. Results are shown below in Tables 2 and 3.
1. Sawant, A. D., M. Gabriel, M. S. Mayo, and D. G. Ahearn. 1991. Radioopacity additives in silicone stent materials reduce in vitro bacterial adherence. Curr. Micorbiol. 22:285-292.
2. Gabriel, M. M., A. D. Sawant, R. B. Simmons, and D. G. Ahearn. 1995. Effects of sliver on adherence of bacteria to urinary catheter: in vitro studies. Curr. Microbio. 30:17-22.

**Table 2**

| Stk CFU = 3.00E+08 | | Pathogen: Pseudomonas aeruginsoa | | | |
|---|---|---|---|---|---|
| DPM | 8699.098 | 3755.142 | 877.6115 | 450.2213 | 122.62 |
| Dilution | 10 | 20 | 100 | 200 | 1000 |
| CFU | 3.00E+07 | 1.50E+07 | 3.00E+06 | 1.50E+06 | 3.00E+05 |
| | | | | | |
| Raw DPM | | | | | |
| | Polymer JR | Control | | | |
| 1 | 917.8193 | 10578.86 | | | |
| 2 | 585.1515 | 5786.323 | | | |
| 3 | 765.0442 | 8762.246 | | | |
| 4 | 625.8184 | 6927.713 | | | |
| 5 | 642.8296 | 5323.255 | | | |
| Bkgd | 30.61751 | 27.33187 | | | |
| surface area (mm²)= | 400 | 400 | | | |
| | | | | | |
| CFU/mm² | PJR | Control | 0 | 0 | 0 |
| 1 | 8.35E+03 | 9.28E+04 | | | |
| 2 | 5.44E+03 | 5.09E+04 | | | |
| 3 | 7.01E+03 | 7.69E+04 | | | |
| 4 | 5.79E+03 | 6.09E+04 | | | |
| 5 | 5.94E+03 | 4.69E+04 | | | |
| Average | 6.51E+03 | 6.57E+04 | | | |
| s.d | 1.18E+03 | 1.91E+04 | | | |

**Table 3**

| Stk CFU = 2.61E+08 | | Pathogen: Pseudomonas aeruginosa | | | |
|---|---|---|---|---|---|
| DPM | 16404.14 | 8107.115 | 2135.891 | 1073.508 | 317.0662 |
| Dilution | 10 | 20 | 100 | 200 | 1000 |
| CFU | 2.61E+07 | 1.31E+07 | 2.61E+06 | 1.31E+06 | 2.61E+05 |
| | | | | | |
| raw DPM | | | | | |
| | | 0.1%PJR 30M | Control | | |
| 1 | | 1924.259 | 13797.67 | | |
| 2 | | 1871.535 | 17582.35 | | |
| 3 | | 4807.021 | 14739.51 | | |
| 4 | | 2332.487 | 24513.43 | | |
| 5 | | 1603.531 | 29132.6 | | |
| Bkgd | | 75 | 75 | | |
| surface area (mm²)= | | 400 | 400 | | |
| | | | | | |
| CFU/mm² | 0 | 0.1%PJR 30M | CW 1.5 | 0 | 0 |
| 1 | | 6.39E+03 | 5.46E+04 | | |
| 2 | | 6.18E+03 | 7.00E+04 | | |
| 3 | | 1.81E+04 | 5.85E+04 | | |
| 4 | | 8.05E+03 | 9.82E+04 | | |
| 5 | | 5.09E+03 | 1.17E+05 | | |
| Average | | 8.77E+03 | 7.96E+04 | | |
| s.d | | 5.33E+03 | 2.69E+04 | | |

## Claims

1. Use of a cationic polysaccharide for inhibiting adhesion of bacteria to the surface of a biomedical device comprising binding said cationic polysaccharide to the surface of the biomedical device.

2. The use as claimed in Claim 1 further comprising treating the surface of the biomedical device to provide a net anionic charge on said surface before contacting the surface with the cationic polysaccharide.

3. The use as claimed in Claim 1 in which the surface of said biomedical device carries a net anionic surface charge and the method includes no intermediate treatment step to modify the surface charge before binding the polysaccharide to the surface of the biomedical device.

4. The use as claimed in Claim 2 in which the surface treating step further comprises contacting said surface with a linking agent.

5. The use as claimed in any preceding claim in which the binding step further comprises retaining the cationic polysaccharide on the surface of said biomedical device through at least one selected from ionic interactions, hydrogen-bonded interactions, hydrophobic interactions and covalent interactions.

6. The use as claimed in Claim 5 in which ionic interactions are between oppositely charged ionic groups between the biomedical device and an aqueous solution containing the cationic polysaccharide.

7. The use as claimed in Claim 6 in which the negative charge on the biomedical device is derived from at least one selected from carboxylate groups, sulfonate groups, phosphate groups and phosphonate groups.

8. The use as claimed in Claim 6 or Claim 7 in which the cationic charge on the cationic polysaacharide is derived from at least one selected from quaternary ammonium groups, sulfonium groups, phosphonium groups.

9. The use as claimed in Claim 5 in which the hydrogen-bonding interactions occur between hydrogen-bond accepting surfaces and hydrogen-bond donating solutions, or through hydrogen-bond donating surfaces and hydrogen-bond accepting solutions.

10. The use as claimed in Claim 9 in which the hydrogen-bond accepting groups are selected from pyrrolidine groups, N,N-disubstituted acrylamide groups and polyether groups.

11. The use as claimed in Claim 10 in which polyether groups are poly(ethylene glycol) or poly(ethylene oxide).

12. The use as claimed in any one of Claims 9 to 11 in which the hydrogen-donating groups are selected from the group consisting of carboxylic acids, phosphoric acids, phosphonic acids and phenolic groups.

13. The use as claimed in Claim 5 in which the hydrophobic interactions occur through hydrophobic sites on the biomaterial surface interacting with hydrophobic groups on the cationic polysaccharide.

14. The use as claimed in Claim 5 in which the covalent interactions exist between the biomaterials surface and the water-soluble cationic polysaccharide such that the cationic polysaccharide is bound to the biomaterial surface.

15. The use as claimed in any preceding claim in which the biomedical device is an ophthalmic lens selected from contact lenses, anterior chamber lenses, posterior chamber lenses, intraocular lenses and corneal inlays.

16. The use as claimed in Claim 15 in which the contact lens is an extended-wear contact lens suitable for periods of continuous wear for about 7 to about 30 days.

17. The use as claimed in any preceding claim in which the biomedical device is a silicone hydrogel material.

18. The use as claimed in any preceding claim in which the cationic polysaccharide is selected from cationic cellulose cationic starch, cationic dextran, cationic chitosan, cationic locust bean gum, cationic gum tragacanth, cationic curdlan, cationic pullulan and cationic scleroglucan.

19. Use of a cationic polysaccharide for the manufacture of a composition for inhibiting adhesion of bacteria to the surface of a biomedical device.

## Patentansprüche

1. Verwendung eines kationischen Polysaccharids zur Inhibierung der Anhaftung von Bakterien an der Oberfläche einer biomedizinischen Vorrichtung. umfassend ein Binden dieses kationischen Polysaccharids an der Oberfläche der biomedizinischen Vorrichtung.

2. Verwendung wie in Anspruch 1 beansprucht, umfassend darüber hinaus die Behandlung der Oberfläche der biomedizinischen Vorrichtung, um eine anionische Netto-Ladung auf der Oberfläche bereitzustellen, bevor die Oberfläche mit dem kationischen Polysaccharid in Kontakt gebracht wird.

3. Verwendung wie in Anspruch 1 beansprucht, worin die Oberfläche der biomedizinischen Vorrichtung eine anionische Netto-Oberflächenladung trägt, und das Verfahren keinen Zwischen-Behandlungsschritt einschließt, um die Oberflächenladung zu modifizieren, bevor das Polysaccharid an der Oberfläche der biomedizinischen Vorrichtung gebunden wird.

4. Verwendung wie in Anspruch 2 beansprucht, worin der Oberflächen-Behandlungsschritt darüber hinaus das In-Kontakt-Bringen der Oberfläche mit einem Bindungsmittel umfasst.

5. Verwendung wie in einem der vorangehenden Ansprüche beansprucht, worin der Bindungsschritt darüber hinaus das Halten des kationischen Polysaccharids an der Oberfläche der biomedizinischen Vorrichtung durch wenigstens eine Wechselwirkung, die ausgewählt ist aus ionischen Wechselwirkungen, Wasserstoff-Bindungs-Wechselwirkungen, hydrophoben Wechselwirkungen und kovalenten Wechselwirkungen, umfaßt.

6. Verwendung wie in Anspruch 5 beansprucht, worin die ionischen Wechselwirkungen zwischen entgegengesetzt geladenen ionischen Gruppen zwischen der biomedizinischen Vorrichtung und einer wässrigen Lösung, die das kationische Polysaccharid enthält, auftreten.

7. Verwendung wie in Anspruch 6 beansprucht, worin die negative Ladung an der biomedizinischen Vorrichtung abgeleitet ist von wenigstens einer Gruppe, die ausgewählt ist aus Carboxylat-Gruppen, Sulfonat-Gruppen, Phosphat-Gruppen und Phosphonat-Gruppen.

8. Verwendung wie in Anspruch 6 oder Anspruch 7 beansprucht, worin die kationische Ladung an dem kationischen Polysaccharid abgeleitet ist von wenigstens einer Gruppe, die ausgewählt ist aus quaternären Ammonium-Gruppen, Sulfonium-Gruppen, Phosphonium-Gruppen.

9. Verwendung wie in Anspruch 5 beansprucht, worin die Wasserstoff-Bindungs-Wechselwirkungen zwischen Wasserstoff-Bindungs-Acceptor-Oberflächen und Wasserstoff-Bindungs-Donor-Lösungen oder zwischen Wasserstoff-Bindungs-Donor-Oberflächen und Wasserstoff-Bindungs-Acceptor-Lösungen stattfinden.

10. Verwendung wie in Anspruch 9 beansprucht, worin die Wasserstoff-Bindungs-Aeceptor-Gruppen ausgewählt sind aus Pyrrolidon-Gruppen, N,N-disubstituierten Acrylamid-Gruppen und Polyether-Gruppen.

11. Verwendung wie in Anspruch 10 beansprucht, worin die Polyether-Gruppen Polyethylenglycol oder Polyethylenoxid sind.

12. Verwendung wie in einem der Ansprüche 9 bis 11 beansprucht, worin die Wasserstoff-Donor-Gruppen ausgewählt sind aus der Gruppe, die besteht aus Carbonsäuren, Phosphorsäuren, Phosphonsäuren und Phenol-Gruppen.

13. Verwendung wie in Anspruch 5 beansprucht, worin die hydrophoben Wechselwirkungen durch die hydrophoben Stellen an der Biomaterial-Oberflächc stattfinden, die mit den hydrophoben Gruppen an dem kationischen Polysaccharid wechselwirken.

14. Verwendung wie in Anspruch 5 beansprucht, worin die kovalenten Wechselwirkungen zwischen der Biomaterial-Oberfläche und dem wasserlöslichen kationischen Polysaccharid existieren, so dass das kationische Polysaccharid an der Biomaterial-Oberfläche gebunden wird.

15. Verwendung wie in einem der vorangehenden Ansprüche beansprucht, worin die biomedizinische Vorrichtung eine ophthalmische Linse ist, die ausgewählt ist aus Kontaktlinsen, vordere Augenkammer-Linsen, hintere Augenkammer-Linsen, intraoculare Linsen und Hoxnhaut-Inlays.

16. Verwendung wie in Anspruch 15 beansprucht, worin die Kontaktlinse eine Kontaktlinse zum verlängerten Tragen ist, die für Zeiträume eines kontinuierlichen Tragens von etwa 7 bis etwa 30 Tagen geeignet ist.

17. Verwendung wie in einem der vorangehenden Ansprüche beansprucht, worin die biomedizinische Vorrichtung ein Silicon-Hydrogel-Material ist.

18. Verwendung wie in einem der vorangehenden Ansprüche beansprucht, worin das kationische Polysaccharid ausgewählt ist aus kationischer Cellulose, kationischer Stärke, kationischem Dextran, kationischem Chitosan, kationischem Johannisbrotkernmehl, kationischen Trngant-Gummi, kationischem Kurdlan, kationischem Pullulan und kationischem Skleroglucan.

19. Verwendung eines kationischen Polysaccharids für die Herstellung einer Zusammensetzungen zur Inhibierung der Anhaftung von Bakterien an der Oberfläche einer biomedizinischen Vorrichtung.

## Revendications

1. Utilisation d'un polysaccharide cationique pour empêcher l'adhérence de bactéries à la surface d'un dispositif biomédical comprenant la fixation dudit polysaccharide cationique à la surface du dispositif biomédical.

2. Utilisation suivant la revendication 1, comprenant de plus le traitement de la surface du dispositif biomédical pour obtenir une charge anionique globale sur ladite surface avant la mise en contact de la surface avec la polysaccharide cationique.

3. Utilisation sulvant la revendication 1, dans laquelle la surface du dispositif biomédical précité porte une charge de surface anionique globale et le procédé ne comprend pas d'étape de traitement Intermédiaire pour modifier la charge de surface avant la fixation du polysaccharide à la surface du dispositif biomédical.

4. Utilisation suivant la revendication 2. dans laquelle l'étape de traitement de la surface comprend de plus la mise en contact de ladite surface avec un agent de liaison.

5. Utilisation suivant l'une quelconque des revendications précédentes, dans laquelle l'étape de fixation comprend de plus le maintien du polysaccharide cationique à la surface du dispositif biomédical précité grâce à au moins une interaction choisie parmi les interactions ioniques, les Interactions à liaison d'hydrogène, les interactions hydrophobes et les Interactions covalentes.

6. Utilisation suivant la revendication 5, dans laquelle il y a des Interactions ioniques entre des groupes ioniques chargés de signes opposés entre la dispositif biomédical et une solution aqueuse contenant le polysaccharide cationique.

7. Utilisation suivant la revendication 6, dans laquelle la charge négative sur le dispositif biomédical provient d'au moins un groupe choisi parmi les groupes carboxylate, les groupes sulfonate, les groupes phosphate et les groupes phosphonate.

8. Utilisation suivant l'un ou l'autre des revendications 6 et 7, dans laquelle la charge cationique sur le polysaccharide cationique provient d'au moins un groupe choisi parmi les groupes ammonium quaternaire, les groupes sulfonium, les groupes phosphonium.

9. Utilisation suivant la revendication 5, dans laquelle les interactions à liaison d'hydrogène se produisent entre des surfaces acceptant des liaisons d'hydrogène et des solutions donnant des liaisons d'hydrogène, ou par l'intermédiaire de surfaces donnant des liaisons d'hydrogène et de solutions acceptant des liaisons d'hydrogène.

10. Utilisation suivant la revendication 9, dans laquelle les groupes acceptant des liaisons d'hydrogène sont choisis parmi les groupes pymalldine. les groupes acrylamlde N,N-disubstitués et les groupes polyéther.

11. Utilisation suivant la revendication 10, dans laquelle les groupes polyéther sont du poly(éthylène glycol) ou du poly(oxyde d'éthytène).

12. Utilisation suivant l'une quelconque des revendications 9 à 11, dans laquelle les groupes donneurs d'hydrogène sont choisis dans le groupe comprenant las acides carboxyliques, les acides phosphoriques, les acides phosphoniques et les groupes phénoliques.

13. Utilisation suivant la revendication 5, dans laquelle les interactions hydrophobes se produisant par l'intermédiaire de sites hydrophobes à la surface du biomatériau interagissant avec des groupes hydrophobes sur le polysaccharide catlonique.

14. Utilisation suivant la revendication 5, dans laquelle les Interactions covalentes existent antre la surface de biomatériaux et le polysaccharide eationique soluble dans l'eau de telle sorte que le polysaccharide cationique soit fixé à la surface du biomatériau.

15. Utilisation suivant l'une quelconque des revendications précédentes, dans laquelle le dispositif biomédical est une lentille ophtalmique choisie parmi les lentilles de contact, les lentilles de chambre antérieure, les lentilles de chambre postérieure, les lentilles intraoculaires et les incrustations coméennes.

16. uulisabon suivant la revendication 15, dans Laquelle la lentille de contact est une lentille de contact de port prolongé convenant pour des périodes de port continu d'environ 7 à environ 30 jours.

17. Utilisation suivant l'une quelconque des revendications précédentes, dans laquelle le dispositif biomédical est une matière d'hydrogel de silicone.

18. Utilisation suivant l'une quelconque des revendications précédentes, dans laquelle le polysaccharide cationique est choisi parmi la cellulose cationique, l'amidon cationique, le dextrane cationique, le chitosane cationique, la gomme de caroube cationique, la gomme adragante cationique, le curdlane cationique, le pullulane cationique et le scléroglucane cationique.

19. Utilisation d'un polysaccharide cationique pour la fabrication d'une composition destinée à empêcher l'adhérence de bactéries à la surface d'un dispositif biomédical.
